# EUROPEAN PATENT SPECIFICATION

(11) **EP 3 318 183 B1**
(45) Date of publication and mention of the grant of the patent: **27.01.2021**
(21) Application number: 15896915.4
(22) Date of filing: 10.09.2015
(51) Int. Cl.: A61B 7/04, A61B 5/0225

(54) **AUXILIARY DEVICE FOR BLOOD PRESSURE MEASUREMENT AND BLOOD PRESSURE MEASURING EQUIPMENT**
HILFSVORRICHTUNG ZUR BLUTDRUCKMESSUNG UND BLUTDRUCKMESSEINRICHTUNG
DISPOSITIF AUXILIAIRE DE MESURE DE LA PRESSION ARTÉRIELLE ET ÉQUIPEMENT DE MESURE DE LA PRESSION ARTÉRIELLE

(30) Priority: 02.07.2015 CN 201510383288
(43) Date of publication of application: 09.05.2018
(73) Proprietor: Zhao, Junfeng, Hongkou, Shanghai 200080 (CN)
(72) Inventor: Zhao, Junfeng, Hongkou, Shanghai 200080 (CN)
(74) Representative: Lock, Graham James
(86) International application number: PCT/CN2015/089367
(87) International publication number: WO 2017/000385

(56) References cited:
- CN-A- 1 302 585
- CN-A- 101 248 990
- CN-A- 101 385 642
- CN-A- 101 664 307
- CN-U- 203 074 686
- KR-B1- 101 313 496

## Description

### Field of the Invention

The present invention relates to the field of blood pressure monitoring, and in particular, to an auxiliary device for blood pressure measurement and blood pressure measuring equipment.

### Description of the Prior art

Currently, sphygmomanometers available on the market are mainly based on two measurement methods: an auscultatory method and an oscillometric method.

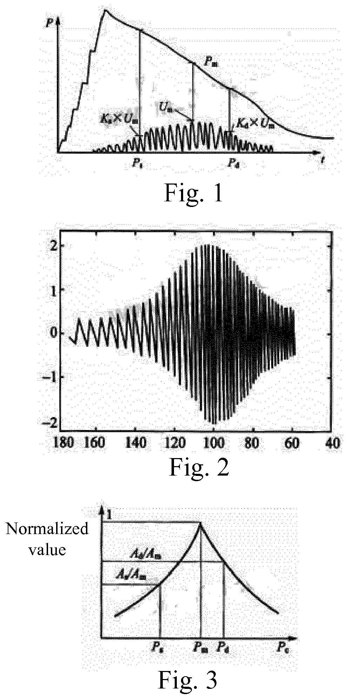

The auscultatory method is the main method used in clinical medication. Mercury sphygmomanometers used by doctors are designed according to the auscultatory method. The auscultatory method is to determine a blood pressure value by using a stethoscope to hear a flowing sound of the blood in the brachial artery with reference to a corresponding pressure when a cuff bladder of the sphygmomanometer is deflated, as shown in FIG. 1. When a pulsating sound is heard in the stethoscope for the first time, the height of the mercury column is the systolic pressure. When the pulsating sound suddenly becomes weak or cannot be heard, the height of the mercury column is the diastolic pressure. For its high measurement accuracy, the auscultatory method is medically referred to as a gold standard for blood pressure measurement. Generally, studies on the measurement accuracy of sphygmomanometers of other types are carried out in comparison with the auscultatory method using a mercury sphygmomanometer. However, the application of the auscultatory method to an electronic sphygmomanometer still has limitations. This is mainly because the capability of electronic devices to distinguish sounds is weaker than human ears in some respects. The auscultatory method requires determining the strength of sound (strength of the pulsating sound) and the frequency of sound (whether the sound is a pulsating sound or noise). As shown in FIG. 2, an electronic sphygmomanometer using the auscultatory method is unpopular due to high design difficulty and costs, and has poor anti-noise performance. Although the mercury sphygmomanometer (or other non-mercury media) using the auscultatory method is convenient, it is difficult for a user to measure his/her blood pressure by himself, and professional training is needed, because ears and eyes need to be highly synchronized during measurement so as to capture the sound change and corresponding pressure value. In addition, data cannot be automatically recorded.

The oscillometric method is to determine the blood pressure value by measuring the amplitude of a pulse vibration wave with reference to a corresponding cuff pressure. Since the principle of the oscillometric method is easy to implement, electronic sphygmomanometers that use the oscillometric method to measure the blood pressure are mainstream products on the market. For example, most Omron electronic sphygmomanometers use the oscillometric method. Many studies have found through comparison that the oscillometric method sometimes has very poor accuracy. According to a report from Centers for Disease Control and Prevention of the United States, the measurement deviation of an Omron sphygmomanometer may be up to 16 mmHg. The low accuracy of the oscillometric method may be due to its measurement principle. In the auscultatory method, during the deflation of the cuff bladder, the blood cannot flow before the pressure decreases to the systolic pressure, so that the user cannot hear the pulsating sound of blood flow. However, in the oscillometric method, before the blood pressure decreases to the systolic pressure, the vibration wave still exists, though with relatively small amplitude. During the deflation of the cuff or the decrease in pressure of the bladder, the changing amplitude of the vibration wave is continuous before and after the systolic pressure and the diastolic pressure. This is different from the auscultatory method where no sound can be heard before a pulsating sound is produced. Therefore, the vibration method can only establish an empirical association between the changing amplitude of the vibration wave and the blood pressure value by using a large number of individual samples. Such an average association established based on a large number of individual samples may not be effective when applied to a certain individual, resulting in that the error of blood pressure measurement is large for a certain proportion of population. The error is due to the deficiencies of the measurement principle, not the quality of the sphygmomanometer.

An amplitude coefficient method, or referred to as 'normalization method', is commonly used in the oscillometric method, as shown in FIG. 3. According to this method, normalization by comparing an amplitude value of a pulse wave vibration signal with a maximum amplitude value of the signal, and identifies the systolic pressure and the diastolic pressure by determining a normalization coefficient of the systolic pressure and the diastolic pressure, as shown in FIG. 3. As is the amplitude of the pulse wave corresponding to the systolic pressure. Am is the amplitude of the pulse wave corresponding to a mean pressure. Ad is the amplitude of the pulse wave corresponding to the diastolic blood pressure. As/Am is a normalized value of a systolic pressure Ps. Ad/Am is a normalized value of a diastolic pressure Pd. Pc is a cuff pressure. Horizontal coordinates represent that the pressure in the cuff continuously decreases during deflation. As/Am=C1, and Ad/Am=C2, respectively corresponding to the positions of the systolic pressure and the diastolic pressure. According to a measured amplitude value of the pulse wave and a corresponding static pressure, the systolic pressure Ps, the diastolic pressure Pd, and the mean pressure Pm can be obtained. Generally, an amplitude coefficient of the systolic pressure is 0.46 to 0.64 and an amplitude coefficient of the diastolic pressure is 0.43 to 0.73. (from http://www.eccn.com/design_2011030416272188.htm). Because different sphygmomanometer manufacturers may use different amplitude coefficients, their measurement value may also differ greatly even if they all use a same oscillometric method. Each individual being measured may correspond to a different amplitude coefficient. However, the sphygmomanometer does not use different coefficients for different people.

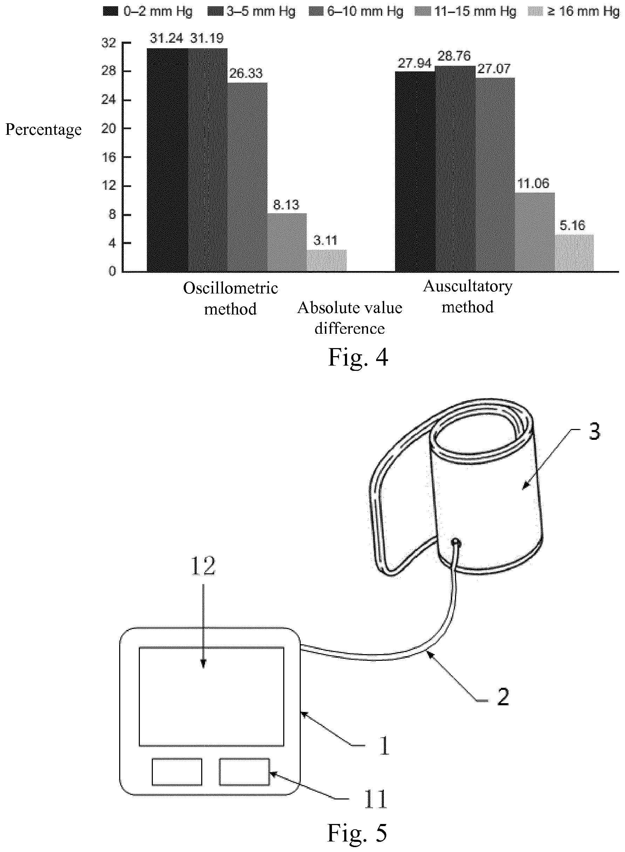

For different people, the measurement results of the electronic sphygmomanometer may be different from those obtained by doctors using the auscultatory method. Statistics shows that the measurement deviation of about 40% people may reach 5 mmHg or more, and the measurement deviation of about 15-20% people may reach 10 mmHg or more. This difference will affect the diagnosis and treatment of hypertension. In addition, because different electronic sphygmomanometer manufacturers use different algorithms, the measurement results of different sphygmomanometer manufacturers for a same person may differ greatly. It is a big challenge for people whose blood pressure needs to be measured to determine the accuracy of the electronic sphygmomanometer. Medically, a user generally brings his/her own electronic sphygmomanometer to a doctor for comparison. Such an operation is inconvenient. In addition, because the blood pressure of a human body is fluctuant, when a reading of the electronic sphygmomanometer is different from a reading measured by the doctor, interference is generated and the determination of the accuracy of the electronic sphygmomanometer is affected. As shown in FIG 4, to determine whether an electronic sphygmomanometer can accurately measure the blood pressure of a particular user, two methods (the oscillometric method used by the electronic sphygmomanometer and the auscultatory method used by the doctor) need to be separately used to measure the blood pressure of the particular user. Then, the measurement results are recorded, and a difference between the measurement results is calculated.

Currently, sphygmomanometers designed using the two methods collect data and perform calculation by themselves, and present only the result to the user in a visual or auditory manner. During the blood pressure measurement, either the sphygmomanometer directly determines the blood pressure value, or the user directly determines the blood pressure value with eyes or ears. As a result, no original data is presented to assist the user in determining the blood pressure value.

CN-A-1302585 discloses a computer network type blood pressure detecting instrument with a mercury column, which is composed of a mercury column type blood pressure detecting instrument, a camera, a stethoscope, a microphone, a multi-media computer and local area net or internet, a modem and a remote end computer. The detecting instrument is connected to the stethoscope which has the microphone set before the stethoscope, the microphone is connected to the multi-media computer or the stethoscope is connected directly to the multi-media computer, the camera is set before the detecting instrument, and the camera is connected to the multi-media computer which is connected with the modem through the local area net or internet to the remote end computer.

CN-U-203074686 discloses a network type mercury column type blood pressure detector mainly comprising a mercury column type blood pressure detector body, a stethoscope, a camera, a microphone and a computer, wherein the mercury column type blood pressure detector body is connected to the stethoscope, the microphone is arranged in front of the stethoscope, the stethoscope and the microphone are connected with the computer, the camera is arranged in front of the mercury column type blood pressure detector body, and the camera is connected with the computer. The blood pressure detector is characterized by further comprising a wireless router and an intelligent terminal, wherein the wireless router is in signal connection with the computer, and the intelligent terminal is in signal connection with the wireless router.

CN-A-101248990 discloses a method and device for visual evaluation of the accuracy of an electronic sphygmomanometer based on the Korotkoff Sound method. The method comprises the following steps: measuring the blood pressure of a subject by the Korotkoff Sound method, recording sound signals from a stethoscope during the measurement, recording changes in the pressure of a cuff by using a pressure sensor during the measurement, converting the recorded pressure and sound signals into digital signals, sending the digital signals to a computer that processes the digital signals and displays, respectively, on a display screen, the wave patterns of the cuff pressure signal and the stethoscope sound signal that vary synchronously along the same timeline, calibrating and verifying the systolic pressure and diastolic pressure values measured by the Korotkoff Sound method according to the wave patterns, and using the values as references for accuracy evaluation of the blood pressure as measured by the electronic sphygmomanometer.

### Summary of the Invention

In view of the disadvantages in the prior art, a technical problem to be resolved in the present invention is to provide a technical solution of blood pressure measurement that can enhance the precision and convenience of the blood pressure measurement, so that a patient can independently measure blood pressure and obtain an accurate measurement result.

In one aspect the present invention provides an auxiliary device for blood pressure measurement according to claim 1.

In a preferred embodiment of the present invention, the terminal device is configured to store and synchronously play the pulse sound signal and synchronously display the pressure image signal.

Further, the terminal device includes a storage module, a display module, and a play module. The storage module is configured to store the pulse sound signal and the pressure image signal. The display module is configured to synchronously display the pressure image signal when the storage module stores the pressure image signal. The play module is configured to synchronously play the pulse sound signal when the storage module stores the pulse sound signal. The displaying of the pressure image signal and the playing of the pulse sound signal are synchronous.

In another preferred embodiment of the present invention, the terminal device is configured to store the pulse sound signal and the pressure image signal, and synchronously display the pulse sound data and the pressure image signal.

Further, the terminal device includes a storage module, a processing module, and a display module. The storage module is configured to store the pulse sound signal and the pressure image signal. The processing module is configured to convert the pulse sound signal into the pulse sound data. The display module is configured to synchronously display the pulse sound data and the pressure image signal.

Further, the terminal device includes a storage module, a processing module, an operation module, and a display module. The storage module is configured to store the pulse sound signal and the pressure image signal. The processing module is configured to convert the pulse sound signal into the pulse sound data and convert the pressure image signal into the pressure data. The operation module is configured to obtain the blood pressure value according to the pulse sound data and the pressure data. The display module is configured to display the blood pressure value obtained by the operation module.

Preferably, the terminal device includes a storage module, a processing module, an operation module, and a display module. The storage module is configured to store the pulse sound signal and the pressure image signal. The processing module is configured to convert the pulse sound signal into the pulse sound data and convert the pressure image signal into the pressure data. The operation module is configured to obtain the blood pressure value according to the pulse sound data and the pressure data. The display module is configured to synchronously display the pulse sound signal and the pressure image signal and display the blood pressure value obtained by the operation module.

Further, the image collection part is disposed in the terminal device.

Further, the audio collection part includes an audio receiving part, an audio preprocessing part, and an audio transmission part. The audio preprocessing part is disposed in the audio receiving part, so that the pulse sound signal received by the audio receiving part is transmitted to the terminal device sequentially by using the audio preprocessing part, the audio transmission part, and a connecting part disposed at an end of the audio transmission part.

Preferably, the audio transmission part is an electrical wire.

Further, the audio collection part includes an audio receiving part, an audio preprocessing part, and an audio transmission part. The audio preprocessing part is disposed in the audio transmission part and is located at an end of the audio transmission part, so that the pulse sound signal received by the audio receiving part is transmitted to the audio preprocessing part by using the audio transmission part. The audio preprocessing part transmits the pulse sound signal to the terminal device by using a connecting part disposed at an end of the audio transmission part.

Preferably, the audio transmission part is a hollow pipe.

Further, the end of the audio transmission part is provided with the connecting part, so that the audio collection part can be electrically connected to the terminal device.

The present invention further provides blood pressure measuring equipment, including a sphygmomanometer and the auxiliary device for blood pressure measurement according to any one of the foregoing descriptions. In the auxiliary device for blood pressure measurement in the present invention, the image collection part records a screen output of an electronic sphygmomanometer or a pressure value corresponding to a mercury column on a mercury column sphygmomanometer by taking a video or a picture, so as to obtain a pressure image signal, and the audio collection part obtains a pulse sound of an upper arm, so that blood pressure can be determined by using an auscultatory method according to the recorded pressure value and the sound. Compared with the prior art, the present invention improves the precision and convenience of blood pressure measurement, so that a vast majority of patients can independently measure blood pressure and obtain an accurate measurement result

A working process of the auxiliary device for blood pressure measurement in the present invention includes the following:
(1) When a user uses an electronic sphygmomanometer, an auscultatory sound (Korotkoff's sound), that is, a pulse sound, is collected by using the audio collection part and the pulse sound is recorded by the terminal device.
   A main difference between the oscillometric method and the auscultatory method in blood pressure measurement is that blood pressure is calculated according to pressure fluctuation in the oscillometric method but blood pressure is directly determined according to an auscultatory sound in the auscultatory method. If the sound is recorded by the terminal device when the electronic sphygmomanometer is used for measurement, the blood pressure can be determined according to the sound recorded by the terminal device.
(2) The pressure image signal is shot by using a shooting function of the image collection part, such as a camera, in the terminal device. The pressure image signal or the pressure data is recorded by the terminal device.
   In the auscultatory method, corresponding cuff pressures when an auscultatory sound appears and disappears need to be found to determine the blood pressure. During measurement, the cuff pressure is generally displayed on a screen of the sphygmomanometer in real time. The pressure image signal (that is, an image displaying the pressure data) of the sphygmomanometer during measurement can be shot by using a shooting function of the terminal device. Since the sound is recorded during shooting, the shot pressure image signal that is displayed on the sphygmomanometer and the auscultatory sound recorded by a smartphone are synchronous.
(3) The auscultatory sound is graphically displayed.
   The auscultatory sound may be graphically displayed on a screen of the terminal device to assist in determining positions where the auscultatory sound appears and disappears.
(4) Image identification of a pressure number is implemented by using the terminal device. A pressure value may be directly extracted by using an image identification technology from an image that displays the pressure data and is shot by the image collection part in the terminal device. In this way, a correspondence between the pressure and the sound is obtained.
(5) Manual or automatic determining is performed on the blood pressure value on the terminal device.

For data of the pressure and sound that is recorded by the terminal device, manual or automatic determining may be performed on the blood pressure value.

Technical effects of the present invention include the following:
(1) The audio collection part collects the pulse sound signal, the image collection part collects the pressure image signal, and the terminal device stores and synchronously plays the pulse sound signal and the pressure image signal, so that a user can independently obtain a pressure value according to the pulse sound signal that is heard and the pressure data in the pressure image signal that is seen.
(2) The audio collection part collects the pulse sound signal, the image collection part collects the pressure image signal, and the terminal device stores the pulse sound signal and the pressure image signal, converts the pulse sound signal into the pulse sound data, and synchronously displays the pulse sound data and the pressure image signal, so that a the user can independently obtain a pressure value according to the pulse sound data and the pressure data in the pressure image signal that are seen.
(3) The audio collection part collects the pulse sound signal, the image collection part collects the pressure image signal, and the terminal device stores the pulse sound signal and the pressure image signal, converts the pulse sound signal into the pulse sound data, converts the pressure image signal into the pressure data, and obtains and displays the blood pressure value according to the pulse sound data and the pressure data, so that a user can directly learn the blood pressure value according to a result displayed on the terminal device or independently obtain a pressure value according to the pulse sound data and the pressure data that are seen.
(4) A blood pressure value measured by an ordinary electronic sphygmomanometer is obtained by using the audio collection part and the terminal device according to the auscultatory method. The auscultatory method is a golden standard of noninvasive blood pressure measurement. Therefore, the obtained blood pressure value may be used to evaluate the measurement accuracy of a particular electronic sphygmomanometer on a particular user.
(5) The auxiliary device for blood pressure measurement in the present invention is applicable to an electronic sphygmomanometer, a sphygmomanometer, and a mercury sphygmomanometer taking millimeter of mercury as a unit.

The following further describes the concepts, specific structures, and generated technical effects of the present invention with reference to the accompanying drawings, so as to fully understand the objectives, features, and effects of the present invention.

### Brief Description of the Drawings

FIG. 1 is a diagram of a correspondence between the amplitude of a pulse wave and a cuff pressure in an oscillometric method;
FIG. 2 is a diagram of a small pulse generated in a cuff;
FIG. 3 is a line graph of a normalized value;
FIG. 4 is a control diagram of an absolute value difference between an auscultatory method and an oscillometric method among different blood pressure measuring equipment;
FIG. 5 is a schematic diagram of an upper arm electronic sphygmomanometer according to a specific embodiment of the present invention;
FIG. 6 is a schematic diagram of a smartphone according to a specific embodiment of the present invention;
FIG. 7 is a schematic diagram of an audio collection part according to a specific embodiment of the present invention; and
FIG. 8 is a schematic diagram of an operating interface of an APP according to Embodiment 3 of the present invention.

### Detailed Description of the Preferred Embodiments

### Embodiment 1

This embodiment provides blood pressure measuring equipment, including a sphygmomanometer and an auxiliary device for blood pressure measurement. The auxiliary device for blood pressure measurement in this embodiment is mainly used to resolve a problem that a user cannot obtain original data when using an existing sphygmomanometer product for measurement, and consequently cannot independently measure blood pressure and obtain an accurate measurement result. In this embodiment, an upper arm electronic sphygmomanometer (referring to FIG. 5) is used, including a host 1, an air pipeline 2, and a cuff bladder 3. The host 1 includes a button 11 and a touch display screen 12. However, the present invention is not limited to the upper arm electronic sphygmomanometer. A mercury sphygmomanometer, a wrist electronic sphygmomanometer, and other sphygmomanometers for different measured parts may alternatively be used.

The auxiliary device for blood pressure measurement in this embodiment includes an audio collection part, an image collection part, and a terminal device. The audio collection part is configured to collect a pulse sound signal during the blood pressure measurement and transmit the pulse sound signal to the terminal device; the image collection part is configured to collect a pressure image signal during the blood pressure measurement and transmit the pressure image signal to the terminal device.

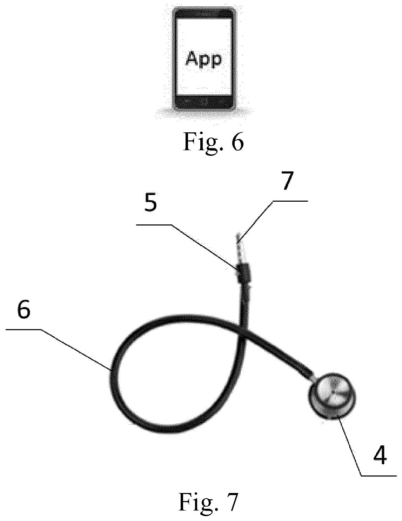

The terminal device in this embodiment is configured to: store the pulse sound signal and the pressure image signal, synchronously play the pulse sound signal, and synchronously display the pressure image signal. Specifically, the terminal device in this embodiment includes a storage module, a display module, and a play module. The storage module is configured to store the pulse sound signal and the pressure image signal. The display module is configured to synchronously display the pressure image signal when the storage module stores the pressure image signal. The play module is configured to synchronously play the pulse sound signal when the storage module stores the pulse sound signal. The displaying of the pressure image signal and the playing of the pulse sound signal are synchronous. The terminal device in this embodiment is a smartphone (referring to FIG. 6). In other embodiments, the terminal device may alternatively be an intelligent device such as a computer, a tablet computer, a wristband, and a watch, or a wearable device.

The image collection part in this embodiment is disposed in the terminal device, and may be a part capable of shooting an image, such as a camera. The audio collection part in this embodiment includes an audio receiving part, an audio preprocessing part, and an audio transmission part. An end of the audio transmission part is provided with a connecting part (for example, a headphone plug), so that the audio collection part can be electrically connected to the terminal device. Specifically, as shown in FIG. 7, the audio collection part in this embodiment is a stethoscope, including an auscultation head 4 and a microphone 5. The microphone 5 is inserted into the audio transmission part 6 and is located at an end of the audio transmission part 6. The headphone plug 7 is directly connected to the microphone 5. In addition, the audio transmission part 6 is a hollow pipe, such as a rubber pipe, so that the pulse sound signal received by the auscultation head 4 is transmitted to the microphone 5 by means of air vibration in the rubber pipe, and then the microphone 5 transmits the pulse sound signal to the terminal device by using the headphone plug.

In other embodiments, the microphone 5 may alternatively be disposed in the auscultation head. In this case, the audio transmission part 6 is an electrical wire, so that the pulse sound signal received by the auscultation head 4 is transmitted to the smartphone sequentially by using the microphone 5, the electrical wire, and the headphone plug 7.

In other embodiments, the stethoscope may alternatively be wirelessly connected to the smartphone. For example, the pulse sound signal is transmitted to the smartphone by using Bluetooth.

### Embodiment 2

This embodiment provides blood pressure measuring equipment, including a sphygmomanometer and an auxiliary device for blood pressure measurement. The auxiliary device for blood pressure measurement in this embodiment is mainly used to resolve a problem that a user cannot obtain original data when using an existing sphygmomanometer product for measurement, and consequently cannot independently measure blood pressure and obtain an accurate measurement result. In this embodiment, an upper arm electronic sphygmomanometer (referring to FIG. 5) is used, including a host 1, an air pipeline 2, and a cuff bladder 3. The host 1 includes a button 11 and a touch display screen 12. However, the present invention is not limited to the upper arm electronic sphygmomanometer. A mercury sphygmomanometer, a wrist electronic sphygmomanometer, and other sphygmomanometers for different measured parts may alternatively be used.

The auxiliary device for blood pressure measurement in this embodiment includes an audio collection part, an image collection part, and a terminal device. The audio collection part is configured to collect a pulse sound signal during the blood pressure measurement and transmit the pulse sound signal to the terminal device; the image collection part is configured to collect a pressure image signal during the blood pressure measurement and transmit the pressure image signal to the terminal device.

The terminal device in this embodiment is configured to: store the pulse sound signal and the pressure image signal, convert the pulse sound signal into pulse sound data, and synchronously display the pulse sound data and the pressure image signal. Specifically, the terminal device in this embodiment includes a storage module, a processing module, and a display module. The storage module is configured to store the pulse sound signal and the pressure image signal. The processing module is configured to convert the pulse sound signal into the pulse sound data. The display module is configured to synchronously display the pulse sound data and the pressure image signal. The terminal device in this embodiment is a smartphone (referring to FIG. 6). In other embodiments, the terminal device may alternatively be an intelligent device such as a computer, a tablet computer, a wristband, and a watch, or a wearable device.

The image collection part in this embodiment is disposed in the terminal device, and may be a part capable of shooting an image, such as a camera. The audio collection part in this embodiment includes an audio receiving part, an audio preprocessing part, and an audio transmission part. An end of the audio transmission part is provided with a connecting part (for example, a headphone plug), so that the audio collection part can be electrically connected to the terminal device. Specifically, as shown in FIG. 7, the audio collection part in this embodiment is a stethoscope, including an auscultation head 4 and a microphone 5. The microphone 5 is inserted into the audio transmission part 6 and is located at an end of the audio transmission part 6. The headphone plug 7 is directly connected to the microphone 5. In addition, the audio transmission part 6 is a hollow pipe, such as a rubber pipe, so that the pulse sound signal received by the auscultation head 4 is transmitted to the microphone 5 by means of air vibration in the rubber pipe, and then the microphone 5 transmits the pulse sound signal to the terminal device by using the headphone plug.

In other embodiments, the microphone 5 may alternatively be disposed in the auscultation head. In this case, the audio transmission part 6 is an electrical wire, so that the pulse sound signal received by the auscultation head 4 is transmitted to the smartphone sequentially by using the microphone 5, the electrical wire, and the headphone plug 7.

In other embodiments, the stethoscope may alternatively be wirelessly connected to the smartphone. For example, the pulse sound signal is transmitted to the smartphone by using Bluetooth.

### Embodiment 3

This embodiment provides blood pressure measuring equipment, including a sphygmomanometer and an auxiliary device for blood pressure measurement. The auxiliary device for blood pressure measurement in this embodiment is mainly used to resolve a problem that a user cannot obtain original data when using an existing sphygmomanometer product for measurement, and consequently cannot independently measure blood pressure and obtain an accurate measurement result. In this embodiment, an upper arm electronic sphygmomanometer (referring to FIG. 5) is used, including a host 1, an air pipeline 2, and a cuff bladder 3. The host 1 includes a button 11 and a touch display screen 12. However, the present invention is not limited to the upper arm electronic sphygmomanometer. A mercury sphygmomanometer, a wrist electronic sphygmomanometer, and other sphygmomanometers for different measured parts may alternatively be used.

The auxiliary device for blood pressure measurement in this embodiment includes an audio collection part, an image collection part, and a terminal device. The audio collection part is configured to collect a pulse sound signal during the blood pressure measurement and transmit the pulse sound signal to the terminal device; the image collection part is configured to collect a pressure image signal during the blood pressure measurement and transmit the pressure image signal to the terminal device.

The terminal device in this embodiment is configured to: store the pulse sound signal and the pressure image signal, convert the pulse sound signal into pulse sound data, convert the pressure image signal into pressure data, and obtain and display a blood pressure value according to the pulse sound data and the pressure data. Specifically, the terminal device in this embodiment includes a storage module, a processing module, an operation module, and a display module. The storage module is configured to store the pulse sound signal and the pressure image signal. The processing module is configured to convert the pulse sound signal into the pulse sound data and convert the pressure image signal into the pressure data. The operation module is configured to obtain the blood pressure value according to the pulse sound data and the pressure data. The display module is configured to display the blood pressure value obtained by the operation module. The terminal device in this embodiment is a smartphone (referring to FIG. 6). In other embodiments, the terminal device may alternatively be an intelligent device such as a computer, a tablet computer, a wristband, and a watch, or a wearable device.

The image collection part in this embodiment is disposed in the terminal device, and may be a part capable of shooting an image, such as a camera. The audio collection part in this embodiment includes an audio receiving part, an audio preprocessing part, and an audio transmission part. An end of the audio transmission part is provided with a connecting part (for example, a headphone plug), so that the audio collection part can be electrically connected to the terminal device. Specifically, as shown in FIG. 7, the audio collection part in this embodiment is a stethoscope, including an auscultation head 4 and a microphone 5. The microphone 5 is inserted into the audio transmission part 6 and is located at an end of the audio transmission part 6. The headphone plug 7 is directly connected to the microphone 5. In addition, the audio transmission part 6 is a hollow pipe, such as a rubber pipe, so that the pulse sound signal received by the auscultation head 4 is transmitted to the microphone 5 by means of air vibration in the rubber pipe, and then the microphone 5 transmits the pulse sound signal to the terminal device by using the headphone plug.

In other embodiments, the microphone 5 may alternatively be disposed in the auscultation head. In this case, the audio transmission part 6 is an electrical wire, so that the pulse sound signal received by the auscultation head 4 is transmitted to the smartphone sequentially by using the microphone 5, the electrical wire, and the headphone plug 7.

In other embodiments, the stethoscope may alternatively be wirelessly connected to the smartphone. For example, the pulse sound signal is transmitted to the smartphone by using Bluetooth.

### Embodiment 4

This embodiment provides blood pressure measuring equipment, including a sphygmomanometer and an auxiliary device for blood pressure measurement. The auxiliary device for blood pressure measurement in this embodiment is mainly used to resolve a problem that a user cannot obtain original data when using an existing sphygmomanometer product for measurement, and consequently cannot independently measure blood pressure and obtain an accurate measurement result. In this embodiment, an upper arm electronic sphygmomanometer (referring to FIG. 5) is used, including a host 1, an air pipeline 2, and a cuff bladder 3. The host 1 includes a button 11 and a touch display screen 12. However, the present invention is not limited to the upper arm electronic sphygmomanometer. A mercury sphygmomanometer, a wrist electronic sphygmomanometer, and other sphygmomanometers for different measured parts may alternatively be used.

The auxiliary device for blood pressure measurement in this embodiment includes an audio collection part, an image collection part, and a terminal device. The audio collection part is configured to collect a pulse sound signal during the blood pressure measurement and transmit the pulse sound signal to the terminal device; the image collection part is configured to collect a pressure image signal during the blood pressure measurement and transmit the pressure image signal to the terminal device.

The terminal device in this embodiment is configured to: store the pulse sound signal and the pressure image signal, convert the pulse sound signal into pulse sound data, convert the pressure image signal into pressure data, and obtain and display a blood pressure value according to the pulse sound data and the pressure data. Specifically, the terminal device in this embodiment includes a storage module, a processing module, an operation module, and a display module. The storage module is configured to store the pulse sound signal and the pressure image signal. The processing module is configured to convert the pulse sound signal into the pulse sound data and convert the pressure image signal into the pressure data. The operation module is configured to obtain the blood pressure value according to the pulse sound data and the pressure data. The display module is configured to synchronously display the pulse sound signal and the pressure image signal and display the blood pressure value obtained by the operation module. The terminal device in this embodiment is a smartphone (referring to FIG. 6). In other embodiments, the terminal device may alternatively be an intelligent device such as a computer, a tablet computer, a wristband, and a watch, or a wearable device.

The image collection part in this embodiment is disposed in the terminal device, and may be a part capable of shooting an image, such as a camera. The audio collection part in this embodiment includes an audio receiving part, an audio preprocessing part, and an audio transmission part. An end of the audio transmission part is provided with a connecting part (for example, a headphone plug), so that the audio collection part can be electrically connected to the terminal device. Specifically, as shown in FIG. 7, the audio collection part in this embodiment is a stethoscope, including an auscultation head 4 and a microphone 5. The microphone 5 is inserted into the audio transmission part 6 and is located at an end of the audio transmission part 6. The headphone plug 7 is directly connected to the microphone 5. In addition, the audio transmission part 6 is a hollow pipe, such as a rubber pipe, so that the pulse sound signal received by the auscultation head 4 is transmitted to the microphone 5 by means of air vibration in the rubber pipe, and then the microphone 5 transmits the pulse sound signal to the terminal device by using the headphone plug.

In other embodiments, the microphone 5 may alternatively be disposed in the auscultation head. In this case, the audio transmission part 6 is an electrical wire, so that the pulse sound signal received by the auscultation head 4 is transmitted to the smartphone sequentially by using the microphone 5, the electrical wire, and the headphone plug 7.

In other embodiments, the stethoscope may alternatively be wirelessly connected to the smartphone. For example, the pulse sound signal is transmitted to the smartphone by using Bluetooth.
Functions such as storage, processing, operation, and displaying of the terminal device in the embodiments may all be implemented by an application (APP) installed in the terminal device.

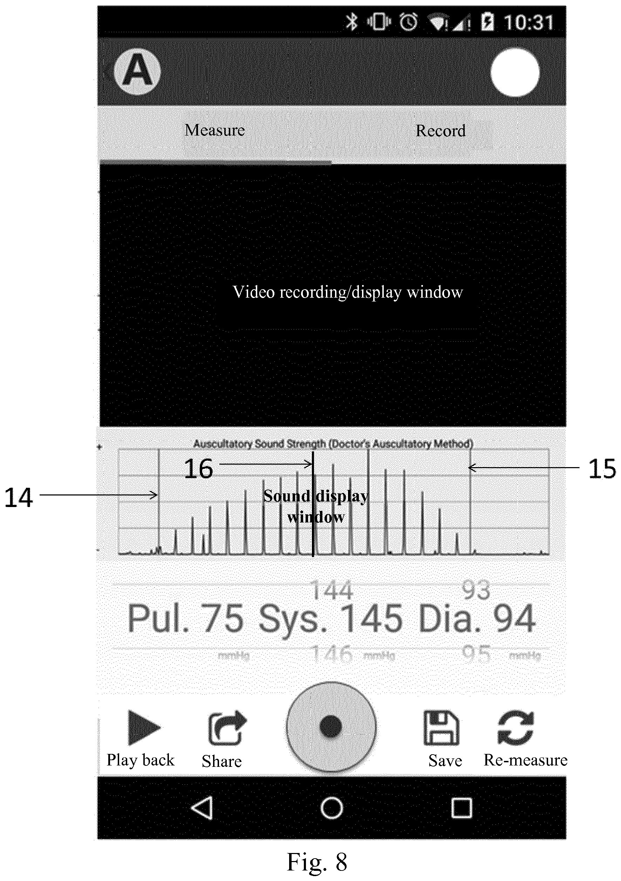

A process of using the blood pressure measuring equipment in the embodiments is as follows:
First, the headphone plug of the stethoscope is plugged into the smartphone during use. A measuring process of the sphygmomanometer is the same as that of an ordinary sphygmomanometer. The sphygmomanometer controls inflation and deflation. The stethoscope is fixed inside the cuff bladder of the sphygmomanometer and is closely attached to a brachial artery to collect an auscultatory sound (a pulse sound). The APP in the smartphone is started. An operating interface of the APP is shown in FIG. 8. The camera on the smartphone is aligned with a display screen of the electronic sphygmomanometer and starts recording an image on the display screen of the sphygmomanometer and a sound in the stethoscope during blood pressure measurement. The APP in the smartphone processes image data and sound data, for example, displays the amplitude of the strength of the sound, obtains a pressure reading according to image identification, and further determines the blood pressure.

The APP in the smartphone may further perform further data analysis: displaying the sound data and the pressure image signal on a same timeline. As shown in FIG. 8, a vertical line 14 on the left of the figure marks a time point that corresponds to a systolic pressure and is determined according to an auscultatory method. A vertical line 15 on the right of the figure marks a time point corresponding to a diastolic pressure. Therefore, images that can correspond to the time point and pressure values displayed on the images are the systolic pressure and the diastolic pressure. When the data is played back, a vertical line 16 in the middle moves with a playing progress of the sound. An amplitude curve of the strength of the sound that the vertical line 16 passes through helps the user more accurately determine the sound heard by ears.

A graphical audio signal of the APP may be in a time domain, or in a frequency domain, or both. The graph is not limited to a line graph, and may be a bar graph or the like. It may be further set that operations such as pausing, continuing, and setting of loop playback between two time points can be performed at any time in a playback process of the sound.

In addition, the user may manually determine, based on a result that the APP graphically displays the pulse sound data and the pressure image signal on a same timeline, the blood pressure value by using the auscultatory method. The APP may alternatively calculate, according to the pressure data, the blood pressure value by using the oscillometric method. Parameters (for example, C1 and C2 in an amplitude parameter method) used in the oscillometric method may be directly adjusted by using an operating interface (not shown). For ordinary users, it may be set, for measurement at a time, to input a blood pressure value determined to be accurate (for example, the blood pressure value manually determined by using the auscultatory method). An exclusive APP is used to compare the input blood pressure value with a blood pressure value determined by using the oscillometric method and adjust the parameters according to a comparison result, so that the parameters can be simply adjusted without professionals. Alternatively, parameters (for example, C1 and C2) used in the oscillometric method may be automatically calibrated based on the blood pressure value determined by using the auscultatory method. This calibration may be set to be periodic, for example, set to automatic calibration after a time period or times. Parameter modification is calibration for an individual situation, so that the measurement precision is generally higher than a direct reading on an ordinary electronic sphygmomanometer. In addition, after the calibration, a relatively accurate blood pressure value can be obtained without using the stethoscope (the auscultatory method is not used to determine the blood pressure value).

Implementations of the foregoing interface functions all fall within the scope that can be implemented in the prior art. In the premise of data sources, manners of data processing and displaying, for example, data correlation analysis, and addition of dynamic elements into images are various, and design or improvement can be further performed according to actual requirements.

During use, the blood pressure measuring equipment in the embodiments is the same as the ordinary electronic sphygmomanometer. Only one click is required to start. The sphygmomanometer automatically performs inflation and deflation to the cuff bladder and synchronously records measured data. After measurement, a blood pressure value determined by using the oscillometric method and/or the auscultatory method is automatically displayed. Alternatively, the recorded pressure image signal (or the pressure data) and sound data may be played back on the terminal device (for example, the smartphone in the embodiments), to manually determine the blood pressure value by using the auscultatory method.

The foregoing describes the preferred specific embodiments of the present invention in detail. It should be understood that a person of ordinary skill in the art can make many modifications and changes according to the concepts of the present invention without creative efforts. Therefore, any technical solution obtained by a person skilled in the art through logical analysis, reasoning, or limited experiments based on the prior art and according to the concepts of the present invention shall all fall within the protection scope determined in the claims.

## Claims

1. An auxiliary device for blood pressure measurement, comprising an audio collection part, an image collection part, and a terminal device, wherein:
the audio collection part is configured to collect a pulse sound signal during the blood pressure measurement and transmit the pulse sound signal to the terminal device;
the image collection part is configured to collect a pressure image signal during the blood pressure measurement and transmit the pressure image signal to the terminal device; and
the terminal device is configured to store the pulse sound signal and the pressure image signal;
**characterized in that**:
the auxiliary device is used for an automatic sphygmomanometer; and
the terminal device is configured to convert the pulse sound signal derived from the automatic sphygmomanometer into pulse sound data, convert the pressure image signal derived from the automatic sphygmomanometer into pressure data, obtain a blood pressure value according to the pulse sound data and the pressure data by using an auscultatory method, and display the blood pressure.

2. The auxiliary device for blood pressure measurement according to claim 1, wherein the terminal device is configured to store the pulse sound signal and the pressure image signal, synchronously play the pulse sound signal, and synchronously display the pressure image signal.

3. The auxiliary device for blood pressure measurement according to claim 2, wherein the terminal device comprises a storage module, a display module, and a play module, wherein the storage module is configured to store the pulse sound signal and the pressure image signal, the display module is configured to synchronously display the pressure image signal when the storage module stores the pressure image signal, the play module is configured to synchronously play the pulse sound signal when the storage module stores the pulse sound signal, and the displaying of the pressure image signal and the playing of the pulse sound signal are synchronous.

4. The auxiliary device for blood pressure measurement according to claim 1, wherein the terminal device is configured to store the pulse sound signal and the pressure image signal, and synchronously display the pulse sound data and the pressure image signal.

5. The auxiliary device for blood pressure measurement according to claim 4, wherein the terminal device comprises a storage module, a processing module, and a display module, wherein the storage module is configured to store the pulse sound signal and the pressure image signal, the processing module is configured to convert the pulse sound signal into the pulse sound data, and the display module is configured to synchronously display the pulse sound data and the pressure image signal.

6. The auxiliary device for blood pressure measurement according to claim 1, wherein the terminal device comprises a storage module, a processing module, an operation module, and a display module, wherein the storage module is configured to store the pulse sound signal and the pressure image signal, the processing module is configured to convert the pulse sound signal into the pulse sound data and convert the pressure image signal into the pressure data, the operation module is configured to obtain the blood pressure value according to the pulse sound data and the pressure data, and the display module is configured to display the blood pressure value obtained by the operation module.

7. The auxiliary device for blood pressure measurement according to claim 1, wherein the terminal device comprises a storage module, a processing module, an operation module, and a display module, wherein the storage module is configured to store the pulse sound signal and the pressure image signal, the processing module is configured to convert the pulse sound signal into the pulse sound data and convert the pressure image signal into the pressure data, the operation module is configured to obtain the blood pressure value according to the pulse sound data and the pressure data, and the display module is configured to synchronously display the pulse sound signal and the pressure image signal, and display the blood pressure value obtained by the operation module.

8. The auxiliary device for blood pressure measurement according to any one of claims 1 to 7, wherein the image collection part is disposed in the terminal device.

9. The auxiliary device for blood pressure measurement according to any one of claims 1 to 7, wherein the audio collection part comprises an audio receiving part (4), an audio preprocessing part (5), and an audio transmission part (6), and the audio preprocessing part (5) is disposed in the audio receiving part (4), so that the pulse sound signal received by the audio receiving part (4) is transmitted to the terminal device sequentially by using the audio preprocessing part (5), the audio transmission part (6), and a connecting part (7) disposed at an end of the audio transmission part (6).

10. The auxiliary device for blood pressure measurement according to claim 9, wherein the audio transmission part (6) is an electrical wire.

11. The auxiliary device for blood pressure measurement according to any one of claims 1 to 7, wherein the audio collection part comprises an audio receiving part (4), an audio preprocessing part (5), and an audio transmission part (6), wherein the audio preprocessing part (5) is disposed in the audio transmission part (6) and is located at an end of the audio transmission part (6), so that the pulse sound signal received by the audio receiving part (4) is transmitted to the audio preprocessing part (5) by using the audio transmission part (6), and the audio preprocessing part (5) transmits the pulse sound signal to the terminal device by using a connecting part (7) disposed at an end of the audio transmission part (6).

12. The auxiliary device for blood pressure measurement according to claim 11, wherein the audio transmission part (6) is a hollow pipe.

13. The auxiliary device for blood pressure measurement according to claim 9, wherein the end of the audio transmission part (6) is provided with the connecting part (7), so that the audio collection part can be electrically connected to the terminal device.

14. Blood pressure measuring equipment, comprising a sphygmomanometer and the auxiliary device for blood pressure measurement according to any one of claims 1 to 7.

## Patentansprüche

1. Hilfsgerät zur Blutdruckmessung, welches ein Audio-Erfassungsteil, ein Bild-Erfassungsteil, und eine Anschlusseinrichtung umfasst, worin:
das Audio-Erfassungsteil ausgestaltet ist, während der Blutdruckmessung ein Pulsgeräuschsignal zu erfassen und das Pulsgeräuschsignal an die Anschlusseinrichtung zu übertragen;
das Bild-Erfassungsteil ausgestaltet ist, während der Blutdruckmessung ein Blutdruckbildsignal zu erfassen und das Blutdruckbildsignal an die Anschlusseinrichtung zu übertragen; und
die Anschlusseinrichtung ausgestaltet ist, das Pulsgeräuschsignal und das Blutdruckbildsignal zu speichern;
**dadurch gekennzeichnet, dass**:
das Hilfsgerät bei einem automatischen Sphygmomanometer verwendet wird; und
die Anschlusseinrichtung ausgestaltet ist, das von dem automatischen Sphygmomanometer abgeleitete Pulsgeräuschsignal in Pulsgeräuschdaten zu wandeln, das von dem automatischen Sphygmomanometer abgeleitete Blutdruckbildsignal in Druckdaten zu wandeln, durch Verwenden eines Auskultationsverfahrens einen Blutdruckwert entsprechend den Pulsgeräuschdaten und den Druckdaten zu erhalten, und den Blutdruck anzuzeigen.

2. Hilfsgerät zur Blutdruckmessung nach Anspruch 1, worin die Anschlusseinrichtung ausgestaltet ist, das Pulsgeräuschsignal und das Blutdruckbildsignal zu speichern, das Pulsgeräuschsignal zeitgleich abzuspielen, und das Blutdruckbildsignal zeitgleich abzuspielen.

3. Hilfsgerät zur Blutdruckmessung nach Anspruch 2, worin die Anschlusseinrichtung ein Speichermodul, ein Anzeigemodul, und ein Abspielmodul umfasst, worin das Speichermodul ausgestaltet ist, das Pulsgeräuschsignal und das Blutdruckbildsignal zu speichern, das Anzeigemodul ausgestaltet ist, das Druckbild zeitgleich anzuzeigen, wenn das Speichermodul das Blutdruckbildsignal speichert, das Abspielmodul ausgestaltet ist, das Pulsgeräuschsignal zeitgleich abzuspielen, wenn das Speichermodul das Pulsgeräuschsignal speichert, und das Anzeigen des Blutdruckbildsignals und das Anzeigen des Pulsgeräuschsignals zeitgleich erfolgen.

4. Hilfsgerät zur Blutdruckmessung nach Anspruch 1, worin die Anschlusseinrichtung ausgestaltet ist, das Pulsgeräuschsignal und das Blutdruckbildsignal zu speichern, und die Pulsgeräuschdaten und das Blutdruckbildsignal zeitgleich anzuzeigen.

5. Hilfsgerät zur Blutdruckmessung nach Anspruch 4, worin die Anschlusseinrichtung ein Speichermodul, ein Prozessormodul, und ein Anzeigemodul umfasst, worin das Speichermodul ausgestaltet ist, das Pulsgeräuschsignal und das Blutdruckbildsignal zu speichern, das Prozessormodul ausgestaltet ist, das Pulsgeräuschsignal in die Pulsgeräuschdaten zu wandeln, und das Anzeigemodul ausgestaltet ist, die Pulsgeräuschdaten und das Blutdruckbildsignal zeitgleich anzuzeigen.

6. Hilfsgerät zur Blutdruckmessung nach Anspruch 1, worin die Anschlusseinrichtung ein Speichermodul, ein Prozessormodul, ein Betriebsmodul, und ein Anzeigemodul umfasst, worin das Speichermodul ausgestaltet ist, das Pulsgeräuschsignal und das Blutdruckbildsignal zu speichern, das Prozessormodul ausgestaltet ist, das Pulsgeräuschsignal in die Pulsgeräuschdaten und das Blutdruckbildsignal in die Druckdaten zu wandeln, das Betriebsmodul ausgestaltet ist, den Blutdruckwert entsprechend den Pulsgeräuschdaten und den Druckdaten zu erhalten, und das Anzeigemodul ausgestaltet ist, den durch das Betriebsmodul erhaltenen Blutdruckwert anzuzeigen.

7. Hilfsgerät zur Blutdruckmessung nach Anspruch 1, worin die Anschlusseinrichtung ein Speichermodul, ein Prozessormodul, ein Betriebsmodul, und ein Anzeigemodul umfasst, worin das Speichermodul ausgestaltet ist, das Pulsgeräuschsignal und das Blutdruckbildsignal zu speichern, das Prozessormodul ausgestaltet ist, das Pulsgeräuschsignal in die Pulsgeräuschdaten und das Blutdruckbildsignal in die Druckdaten zu wandeln, das Betriebsmodul ausgestaltet ist, den Blutdruckwert entsprechend den Pulsgeräuschdaten und den Druckdaten zu erhalten, und das Anzeigemodul ausgestaltet ist, das Pulsgeräuschsignal und das Blutdruckbildsignal zeitgleich anzuzeigen, und den durch das Betriebsmodul erhaltenen Blutdruckwert anzuzeigen.

8. Hilfsgerät zur Blutdruckmessung nach einem der Ansprüche 1 bis 7, worin das Bild-Erfassungsteil in der Anschlusseinrichtung angeordnet ist.

9. Hilfsgerät zur Blutdruckmessung nach einem der Ansprüche 1 bis 7, worin das Audio-Erfassungsteil ein Audio-Empfangsteil (4), ein Audio-Prozessorteil (5), und ein Audio-Übertragungsteil (6) umfasst, und das Audio-Prozessorteil (5) in dem Audio-Empfangsteil (4) vorgesehen ist, so dass das durch das Audio-Empfangsteil (4) empfangene Pulsgeräuschsignal an die Anschlusseinrichtung durch Verwenden des Audio-Prozessorteils (5), des Audio-Übertragungsteils (6), und eines Verbindungsteils (7), das an einem Ende des Audio-Übertragungsteils (6) angeordnet ist, sequentiell übertragen wird.

10. Hilfsgerät zur Blutdruckmessung nach Anspruch 9, worin das Audio-Übertragungsteil (6) ein elektrischer Draht ist.

11. Hilfsgerät zur Blutdruckmessung nach einem der Ansprüche 1 bis 7, worin das Audio-Erfassungsteil ein Audio-Empfangsteil (4), ein Audio-Prozessorteil (5), und ein Audio-Übertragungsteil (6) umfasst, worin das Audio-Prozessorteil (5) in dem Audio-Übertragungsteil (6) vorgesehen ist und an einem Ende des Audio-Übertragungsteils (6) vorgesehen ist, so dass das durch das Audio-Empfangsteil (4) empfangene Pulsgeräuschsignal durch Verwenden des Audio-Übertragungsteils (6) an das Audio-Prozessorteil (5) übertragen wird, und das Audio-Prozessorteil (5) das Pulsgeräuschsignal durch Verwenden eines an einem Ende des Audio-Übertragungsteils (6) vorgesehenen Verbindungsteiles (7) an die Anschlusseinrichtung überträgt.

12. Hilfsgerät zur Blutdruckmessung nach Anspruch 11, worin das Audio-Übertragungsteil (6) eine hohle Röhre ist.

13. Hilfsgerät zur Blutdruckmessung nach Anspruch 9, worin das Ende des Audio-Übertragungsteils (6) mit dem Verbindungsteil (7) versehen ist, so dass das Audio-Erfassungsteil mit der Anschlusseinrichtung elektrisch verbunden werden kann.

14. Blutdruckmessungsausrüstung, welche einen Sphygmomanometer und das Hilfsgerät zur Blutdruckmessung nach einem der Ansprüche 1 bis 7 umfasst.

## Revendications

1. Dispositif auxiliaire de mesure de la pression sanguine, comprenant une partie de collecte audio, une partie de collecte d'image et un dispositif terminal, dans lequel :
la partie de collecte audio est configurée pour collecter un signal sonore de pouls pendant la mesure de la pression sanguine et transmettre le signal sonore de pouls au dispositif terminal ;
la partie de collecte d'image est configurée pour collecter un signal d'image de pression pendant la mesure de la pression sanguine et transmettre le signal d'image de pression au dispositif terminal ; et
le dispositif terminal est configuré pour stocker le signal sonore de pouls et le signal d'image de pression ;
**caractérisé par le fait que** :
le dispositif auxiliaire est utilisé pour un sphygmomanomètre automatique ; et
le dispositif terminal est configuré pour convertir le signal sonore de pouls provenant du sphygmomanomètre automatique en données sonores de pouls, convertir le signal d'image de pression provenant du sphygmomanomètre automatique en données de pression, obtenir une valeur de pression sanguine en fonction des données sonores de pouls et des données de pression par utilisation d'une méthode auscultatoire, et afficher la pression sanguine.

2. Dispositif auxiliaire de mesure de la pression sanguine selon la revendication 1, dans lequel le dispositif terminal est configuré pour stocker le signal sonore de pouls et le signal d'image de pression, lire le signal sonore de pouls de manière synchrone, et afficher le signal d'image de pression de manière synchrone.

3. Dispositif auxiliaire de mesure de la pression sanguine selon la revendication 2, dans lequel le dispositif terminal comprend un module de stockage, un module d'affichage et un module de lecture, le module de stockage étant configuré pour stocker le signal sonore de pouls et le signal d'image de pression, le module d'affichage étant configuré pour afficher le signal d'image de pression de manière synchrone lorsque le module de stockage stocke le signal d'image de pression, le module de lecture étant configuré pour lire le signal sonore de pouls de manière synchrone lorsque le module de stockage stocke le signal sonore de pouls, et l'affichage du signal d'image de pression et la lecture du signal sonore de pouls sont synchrones.

4. Dispositif auxiliaire de mesure de la pression sanguine selon la revendication 1, dans lequel le dispositif terminal est configuré pour stocker le signal sonore de pouls et le signal d'image de pression et afficher les données sonores de pouls et le signal d'image de pression de manière synchrone.

5. Dispositif auxiliaire de mesure de la pression sanguine selon la revendication 4, dans lequel le dispositif terminal comprend un module de stockage, un module de traitement et un module d'affichage, le module de stockage étant configuré pour stocker le signal sonore de pouls et le signal d'image de pression, le module de traitement étant configuré pour convertir le signal sonore de pouls en les données sonores de pouls, et le module d'affichage étant configuré pour afficher les données sonores de pouls et le signal d'image de pression de manière synchrone.

6. Dispositif auxiliaire de mesure de la pression sanguine selon la revendication 1, dans lequel le dispositif terminal comprend un module de stockage, un module de traitement, un module de calcul et un module d'affichage, le module de stockage étant configuré pour stocker le signal sonore de pouls et le signal d'image de pression, le module de traitement étant configuré pour convertir le signal sonore de pouls en les données sonores de pouls et convertir le signal d'image de pression en les données de pression, le module de calcul étant configuré pour obtenir la valeur de pression sanguine en fonction des données sonores de pouls et des données de pression, et le module d'affichage étant configuré pour afficher la valeur de pression sanguine obtenue par le module de calcul.

7. Dispositif auxiliaire de mesure de la pression sanguine selon la revendication 1, dans lequel le dispositif terminal comprend un module de stockage, un module de traitement, un module de calcul et un module d'affichage, le module de stockage étant configuré pour stocker le signal sonore de pouls et le signal d'image de pression, le module de traitement étant configuré pour convertir le signal sonore de pouls en les données sonores de pouls et convertir le signal d'image de pression en les données de pression, le module de calcul étant configuré pour obtenir la valeur de pression artérielle en fonction des données sonores de pouls et des données de pression, et le module d'affichage étant configuré pour afficher le signal sonore de pouls et le signal d'image de pression de manière synchrone et afficher la valeur de pression sanguine obtenue par le module de calcul.

8. Dispositif auxiliaire de mesure de la pression sanguine selon l'une quelconque des revendications 1 à 7, dans lequel la partie de collecte d'image est disposée dans le dispositif terminal.

9. Dispositif auxiliaire de mesure de la pression sanguine selon l'une quelconque des revendications 1 à 7, dans lequel la partie de collecte audio comprend une partie de réception audio (4), une partie de prétraitement audio (5) et une partie de transmission audio (6), et la partie de prétraitement audio (5) est disposée dans la partie de réception audio (4) de telle sorte que le signal sonore de pouls reçu par la partie de réception audio (4) est transmis au dispositif terminal séquentiellement par utilisation de la partie de prétraitement audio (5), de la partie de transmission audio (6) et d'une partie de connexion (7) disposée à une extrémité de la partie de transmission audio (6).

10. Dispositif auxiliaire de mesure de la pression sanguine selon la revendication 9, dans lequel la partie de transmission audio (6) est un fil électrique.

11. Dispositif auxiliaire de mesure de la pression sanguine selon l'une quelconque des revendications 1 à 7, dans lequel la partie de collecte audio comprend une partie de réception audio (4), une partie de prétraitement audio (5) et une partie de transmission audio (6), la partie de prétraitement audio (5) étant disposée dans la partie de transmission audio (6) et étant située à une extrémité de la partie de transmission audio (6), de telle sorte que le signal sonore de pouls reçu par la partie de réception audio (4) est transmis à la partie de prétraitement audio (5) par l'utilisation de la partie de transmission audio (6), et la partie de prétraitement audio (5) transmettant le signal sonore de pouls au dispositif terminal par l'utilisation d'une partie de connexion (7) disposée à une extrémité de la partie de transmission audio (6) .

12. Dispositif auxiliaire de mesure de la pression sanguine selon la revendication 11, dans lequel la partie de transmission audio (6) est un tube creux.

13. Dispositif auxiliaire de mesure de la pression sanguine selon la revendication 9, dans lequel l'extrémité de la partie de transmission audio (6) comprend la partie de connexion (7), de telle sorte que la partie de collecte audio peut être connectée électriquement au dispositif terminal.

14. Équipement de mesure de la pression sanguine, comprenant un sphygmomanomètre et le dispositif auxiliaire de mesure de pression artérielle selon l'une quelconque des revendications 1 à 7.
